# EUROPEAN PATENT APPLICATION

(11) **EP 3 888 662 A1**
(43) Date of publication of application: **06.10.2021**
(21) Application number: 20167832.3
(22) Date of filing: 02.04.2020
(51) Int. Cl.: A61K 31/704, A61K 31/352, A61K 31/56, A61K 31/397, A61K 31/7048, A61K 36/484, A61P 31/14, A24B 15/16

(54) **COMPOUNDS, COMPOSITIONS AND DEVICES FOR USE IN THE TREATMENT OF CORONAVIRUS INFECTIONS**

(71) Applicant: Vossensteyn, 2712 LA Zoetermeer (NL)
(72) Inventor: Steijn, Petrus Hendrikus Jacobus, 2712 LA Zoetermeer (NL); de Vos, Martijn, 2712 LA Zoetermeer (NL)
(74) Representative: Willnegger, Eva

(57) **Abstract**

The present invention provides method of treating a disease associated with coronavirus infection by inhalation of a compound selected from the group of glycyrrhizin, 18a-glycerrhetinic acid, 18β-glycerrhetinic acid, glycyrrhizin derivatives, baicalin, Scutellarin, Hesperetin and nicotianamine. Also provided are compositions and inhalation devices for use in such method.

## Description

The invention concerns a method of treating respiratory viral infections, and compounds and devices for use in such treatment.

Corona viruses (CoVs) form a family of enveloped, positive-sense, single stranded RNA viruses that are able to infect a broad range of vertebrates. They can be found in many birds, especially bats, and mammals and can cause a variety of diseases, of which mild to moderate respiratory tract infections are the most common syndromes. However, in 2002 a novel coronavirus started the outbreak of severe acute respiratory syndrome (SARS) in China, which rapidly spread out through 29 countries in Southeast Asia, resulting in over 8000 confirmed cases and nearly 800 deaths. The virus responsible for this SARS pandemic was identified as SARS-Corona virus (SARS-CoV).

In December 2019 an outbreak of potentially fatal atypical pneumonia, defined as Coronavirus Disease 19 (COVID-19) occurred in Wuhan, China. A distinctive, until then unknown coronavirus was identified to be responsible for the pandemic that followed this outbreak, and was characterized as SARS-CoV-2. This SARS-CoV-2 shares 88% sequence identity with two SARS-like coronaviruses that originated in bats. The genome encodes four major structural proteins: The spike (S) protein, nucleocapsid (N) protein, membrane (M) protein and the envelope (E) protein. The S protein of SARS-CoV-2, responsible for entry into the cell, has a receptor binding motif (RBM) that is largely identical to the other SARS-CoV, suggesting that these viruses use the same host receptor. For SARS-CoV the host receptor has been identified as Angiotensin Converting Enzyme 2 (ACE2) By using structural modeling of the S protein of SARS-CoV-2, a strong interaction of human ACE2 molecules with SARS-CoV-2 is also suggested (Xu et al.2020 "Evolution of the novel coronavirus from the ongoing Wuhan outbreak and modeling of its spike protein for risk of human transmission." Science China Life sciences (1674-7305).doi:https://doi.org/10.1007/s11427-020-1637-6)

ACE2 is a transmembrane metalloperoxydase and is mainly expressed in vascular endothelial and renal tubular cells, but can also be found in the lung, especially in the alveolar type II-pneumocytes. Several independent research groups (have shown, that SARS-CoV-2 utilizes ACE2 as a cellular entry receptor, just like SARS-CoV that was responsible for the 2002 pandemic outbreak of SARS. For entry into the host cell, SARS-CoV uses the S protein, which has to be cleaved before viral and cellular membranes can fuse. This cleavage, termed S-protein priming, occurs by cellular protease, of which transmembrane protease serine 2 is crucial.

The outbreak of COVID-19 and its pandemic spread has already resulted in more casualties and deaths than the 2002 pandemic outbreak of SARS. Hence there is a great need for therapeutic agents that can treat COVID-19.

Licorice is an ancient herb, which has been widely used in traditional Chinese medicine. Three original plants from the family of *Leguminosae, Glycyrrhiza uralensis, Glycyrrhiza inflate,* and *Glycyrrhiza glabra,* are prescribed as licorice. The anti-inflammatory properties of licorice and some of its active components has been described. (Rui Yang et al. 2017. Pharmaceutical Biology, 55(1): 5-18).Radix Glycyrrhizae, a hot water extract of a preparation of licorice, and 18β-glycyrrhetinic acid, one of the active constituents of licorice, have been shown to have antiviral activity against human respiratory syncytial virus (HRSV) by inhibiting viral attachment and internalization. (C. Feng Yeh et al. 2013. J. Ethnopharmacology 148: 466-473) Glycyrrhizin is also used in the treatment of chronic hepatitis, and is relatively non-toxic. It was shown to inhibit viral absorption and penetration of the SARS-CoV *in vitro* in Vero cells. It was found most effective when administered during and after viral adsorption period. (J. Cinatl et al. 2003. The Lancet, Vol 361, June 14, pp. 2045-2046).Recently glycyrrhizin has been shown to bind to ACE2. (Hansen et al. Preprints (www.preprints.org) Posted 30 January 2020)

No treatment or therapeutic agent that could treat COVID-19 has been developed so far. It is an object of the present invention to provide a method that treats diseases associated with coronavirus infections, in particular SARS-associated COVID-19. It is a further object of the invention to provide compounds, compositions comprising these compounds and devices that can be used in such treatment.

The present invention provides a method to treat diseases associated with coronavirus infections, in particular diseases associated with SARS-associated coronavirus infections, more preferably COVID-19, by administration of a compound selected from the group of glycyrrhizin, 18α-glycerrhetinic acid, 18β-glycerrhetinic acid, glycyrrhizin derivatives, baicalin, Scutellarin, Hesperetin and nicotianamine directly into the lungs. By administering the compounds directly into the lung via inhalation, the compounds can inhibit the virus from entering the lung epithelial cells by targeting the ACE2 receptor present in the membrane of the lung cells, thereby attenuating viral infection and onset of the disease.

Although extracts of licorice and its active components have been suggested to have antiviral properties, research is focused on oral application of these compounds. Application of the drugs to treat coronavirus infections of the lungs via inhalation has not been suggested. In view of the rapid pandemic spread that infection with those viruses, in particular SARS-associated coronaviruses can cause, it is expected that traditional oral application of the antiviral compounds will not effectively treat the diseases. Providing the compounds of the invention directly into the lungs has the advantage that the compounds can immediately inhibit the virus from entering the lung epithelial cells by targeting the ACE2 receptor present in the membrane of the lung cells, thereby attenuating viral infection and onset of the disease.

The compounds of the invention can be applied directly into the lungs via inhalation using standard inhalation devices. Of particular interest are inhalation devices that provide the compounds via thermic evaporization. Such devices are also referred to as vaporizers, which vaporize a solution comprising the active compounds. By inhaling the vapor, the active compounds directly enter the lungs where they can effectively display there therapeutic action. Preferably the vaporizer is an electronic vaporizer, more preferably a hand-held electronic vaporizer. The use of a vaporizer, in particular an electronic vaporizer, in the method of the invention has the advantage of creating ultrafine particles through vaporization of the composition comprising the active compounds, thereby targeting the lower airways and alveoli, where expression of ACE2 is prominent. Thus, administration via vaporization has the advantage of providing the active compounds directly to the site of action of the coronavirus, where the compounds can compete with the virus for binding to the ACE2 receptor, thereby inhibiting further infection of the lungs. In view of the low toxicity of the compounds administration of these compounds with a vaporizer will have low to no adverse effects.

Thus in a first aspect, there is provided a compound selected from the group of glycyrrhizin, glycyrrhizin derivatives, 18α-glycerrhetinic acid, 18β-glycerrhetinic acid, baicalin, Scutellarin, Hesperetin and nicotianamine for use in the treatment of diseases associated with coronaviruses via administration in the lungs. Preferably the compound for use according to the invention is selected from glycyrrhizin, 18α-glycerrhetinic acid and 18β-glycerrhetinic acid. More preferably the compound for use according to the invention is glycyrrhizin. The compounds of the invention are preferably used to treat diseases associated with SARS-like coronaviruses, preferably diseases associated with SARS-coronaviruses. Most preferably the compounds of the invention are used to treat COVID-19 by inhalation, in particular inhalation with a vaporizer, preferably an electronic vaporizer.

Glycyrrhizin, 18α-glycerrhetinic acid, and 18β-glycerrhetinic acid are active components that are present in licorice and are commercially available. Derivatives of glycyrrhizin are also suitable for use in the method according to the invention. Baicalin is an active compound that can be obtained from the Chinese medical plant Scutellaria baicalensis Georgi. Scutellarin is an active compound of Chinese Medicine Erigeron breviscapus (Vant.) Hand Mazz. Hesperetin is a biflavonoid compound abundant in Chinese medicine citrus aurantium and Citri Reiculatae Pericarpium. Nicotianamine is a small metal-chelating molecule that is present in higher plants such as soybean. Similar to glycyrrhizin, these compounds have been found to bind to ACE2.

Glycyrrhizin, 18α-glycerrhetinic acid and 18β-glycerrhetinic acid have the advantage that they have a low toxicity, which makes these compounds suitable for administration in the lungs, in particular via inhalation, more particular vaporization.

In a second aspect of the invention, liquid compositions comprising a compound selected from the group of glycyrrhizin, 18α-glycerrhetinic acid,18β-glycerrhetinic acid, glycyrrhizin derivatives, baicalin, Scutellarin, Hesperetin and nicotianamine for use in an inhalation device are provided. These compositions comprises the standard pharmaceutical excipients that are required for administration of aerosols and vapors in the lungs via inhalation. Preferred compositions are liquid aerosol solutions comprising a compound selected from the group of glycyrrhizin, 18α-glycerrhetinic acid,18β-glycerrhetinic acid and glycyrrhizin derivatives. These aerosol solutions are solutions that are particularly suitable for inhalation.

In a preferred embodiment of the invention, the liquid composition comprising the compounds of the invention is an e-liquid composition. E-liquid compositions are solutions that are particularly suitable for use in an electronic vaporizer. Thus the present invention provides for an e-liquid composition comprising a compound selected from the group of glycyrrhizin, 18α-glycerrhetinic acid, 18β-glycerrhetinic acid, glycyrrhizin derivatives baicalin, Scutellarin, Hesperetin and nicotianamine. Preferably the e-liquid composition comprises a compound selected from glycyrrhizin, 18α-glycerrhetinic acid and 18β-glycerrhetinic acid. More preferably the e-liquid composition comprises glycyrrhizin as the active compound. The compositions of the invention as described herein comprise the active compound in a therapeutically effective amount. Preferably the active compounds is present in the composition described herein in a concentration of 1 pg/ml or more to be therapeutically effective.

Generally these e-liquid compositions comprise a carrier to ensure that most of the active ingredients of the composition are evaporated. Suitable carriers are standard carriers that are known in the art of e-liquids such as alcohols e.g. ethanol, glycerol (also known as glycerin), propylene glycol, polyethylene glycol and the like. Preferably the carrier is selected from the group of propylene glycol, glycerin, and mixtures thereof. More preferably the carrier is a mixture of propylene and glycerin. Thus in a preferred embodiment, the present invention provides for an e-liquid composition comprising a compound selected from the group of glycyrrhizin, 18α-glycerrhetinic acid, 18β-glycerrhetinic acid, glycyrrhizin derivatives, baicalin, Scutellarin, Hesperetin and nicotianamine, and a carrier selected from propylene glycol and glycerin. Preferably the e-liquid composition comprises a compound selected from the group of glycyrrhizin, 18α-glycerrhetinic acid, 18β-glycerrhetinic acid and a mixture of propylene glycol and glycerin. More preferably the e-liquid composition comprises glycyrrhizin and a mixture of propylene glycol and glycerin. Preferably the mixture of propylene glycol and glycerin is a mixture of 50% propylene glycol and 50% glycerin.

The solution and e-liquid compositions according to the invention may further comprise additives such as vitamins, minerals, taste enhancers and the like. Preferred additives are vitamins such as vitamin C, and minerals such as selenium and zinc. Suitable taste enhancers can be any taste enhancer that has been approved in the food industry such as fruit- and vegetable flavors and the like. E-liquids having nicotine and licorice have been described. The licorice however is used as a taste enhancer and the active antiviral components present in the licorice are present in very small concentrations of about 0.005 pg/ml or less. Such amounts of licorice and its active components are too low to exert any therapeutic effect. Furthermore, these e-liquids comprising licorice are for use in electronic cigarettes and cigars and usually comprise nicotine. Such e-liquids comprising licorice are therefore not suitable for therapeutic use. The e-liquids of the present invention preferably do not comprise nicotine.

The liquid compositions according to the invention can be prepared using common general knowledge and standard techniques.

The compounds and liquid compositions of the invention can be applied directly into the lungs via inhalation using standard inhalation devices. Preferred inhalation devices are electronic vaporizers. Such electronic vaporizer for example include:
- a housing usually cylindrical, e.g., having about the size and shape of a cigarette or cigar;
- a cartridge or reservoir or pod or container, for holding an e-liquid;
- a thermal atomizer;
- a power source, such as a battery;
- an inhalation mouthpiece.

The vaporizer can be made as a single use disposable device or it may be provided with a removable and disposable cartridge or a refillable cartridge, fitting in a matching recess of the housing and configured to deliver the e-liquid to the mouth piece. The vaporizer may contain one or more sensors and associated circuitry to allow puffing to activate the battery-powered thermal atomizer, which vaporizes the liquid in the cartridge. The user then inhales the resulting aerosol or vapor. Such vaporizers are known in the art. Examples of such vaporizers are disclosed in EP1618803 en US1775947. Thus in a further aspect, inhaling devices are provided which comprise a container that is filed with a liquid composition according to the invention as described herein. Preferably, the inhaling device is a vaporizer, in particular an electronic vaporizer as described herein, wherein the cartridge comprises an e-liquid composition according to the invention as described herein. The vaporizer can be a single use disposable vaporizer or it may be provided with a removable and disposable cartridge or a refillable cartridge.

In yet a further aspect, the invention provides for a container comprising a compound or composition of the invention as described in this application. Preferred containers are cartridges for use in an electronic vaporizer equipped with a removable and disposable cartridge, said cartridges comprising an e-liquid composition according to the invention described herein. Thus in a another aspect, the invention provides for a container comprising a compound or liquid composition according to the invention described herein, said container being suitable for use in a reusable inhalation device, preferably a reusable vaporizer, more preferably a reusable electronic vaporizer as described herein. The invention also provides for a kit of part comprising two or more containers each container comprising a compound or a liquid composition according to the invention as described herein. Preferably the kit comprises two or more cartridges comprising an e-liquid according to the invention as described herein, more preferably disposable cartridges. In another aspect, the invention provides for a kit of parts comprising one or more containers according to the invention described herein and an inhalation device. Preferably the kit comprises one or more cartridges comprising an e-liquid according to the invention described herein and a vaporizer. In a preferred embodiment the kit comprises one or more cartridges comprising an e-liquid according to the invention described herein and an electronic vaporizer equipped with a removable and disposable cartridge. The cartridges of the kit of parts can be used to replace the empty cartridges of said electronic vaporizer.

## Claims

1. A compound selected from the group of glycyrrhizin, 18α-glycerrhetinic acid, 18β-glycerrhetinic acid, glycyrrhizin derivatives, baicalin, Scutellarin, Hesperetin and nicotianamine for use in the treatment of a disease associated with a coronavirus infection by administration in the lungs.

2. The compound for use according to claim 2 wherein the corona virus is a SARS-associated corona virus.

3. The compound for use according to claim 2 wherein the disease is COVID-19.

4. Use of a compound selected from the group of glycyrrhizin, 18α-glycerrhetinic acid, 18β-glycerrhetinic acid, glycyrrhizin derivatives, baicalin, Scutellarin, Hesperetin and nicotianamine in the treatment of a disease associated with coronavirus infection via administration in the lungs.

5. Use according to claim 5 wherein the disease is associated with a SARS-associated coronavirus.

6. Use according to claim 5 or 6 wherein the disease is COVID-19.

7. An aerosol composition for use in an inhalation device, said composition comprising a compound selected from the group of glycyrrhizin, 18α-glycerrhetinic acid, 18β-glycerrhetinic acid, glycyrrhizin derivatives, baicalin, Scutellarin, Hesperetin and nicotianamine, and one or more pharmaceutical aerosol excipients.

8. An e-liquid composition comprising a compound selected from the group of glycyrrhizin, 18α-glycerrhetinic acid, 18β-glycerrhetinic acid, glycyrrhizin derivatives, baicalin, Scutellarin, Hesperetin and nicotianamine.

9. The aerosol composition according to claim 7, or e-liquid according to claim 8, which further comprises an inhalation carrier.

10. The aerosol composition or e-liquid according to claim 9, wherein the carrier is selected from the group of propylene glycol, glycerin and mixtures thereof.

11. The aerosol composition or e-liquid composition according to claim 10, wherein the carrier is a mixture of propylene glycol and glycerin, more preferably a mixture of 50% propylene glycol and 50% glycerin.

12. A container for use in an inhalation or vaporizing device, said container comprising an aerosol composition or e-liquid composition according to any one of the preceding claims.

13. A kit of parts for use in the treatment of a disease associated with a coronavirus infection by administration in the lungs via inhalation, said kit comprising two or more containers according to claim 12.

14. A kit of parts for use in the treatment of a disease associated with a coronavirus infection by administration in the lungs via inhalation, said kit comprising one or more containers according to claim 12 and an inhalation device.

15. A kit of parts according to claim 14, wherein the device is an electronic vaporizer equipped with a removable cartridge, preferably an electronic vaporizer.
